# EUROPEAN PATENT APPLICATION

(11) **EP 4 621 723 A1**
(43) Date of publication of application: **24.09.2025**
(21) Application number: 24165487.0
(22) Date of filing: 22.03.2024
(51) Int. Cl.: G06T 17/00, G06V 20/40, G06V 40/10, G10L 13/00, H04M 3/42, G16H 20/30

(54) **ARTIFICIALLY INTELLIGENT AVATARS OF REAL-WORLD ATHLETES**

(71) Applicant: PlayAI GmbH, 81241 München (DE)
(72) Inventor: Taylor, Samuel, 3131 Melbourne/VIC (AU); Martin, Anja, 81241 Munich (DE)
(74) Representative: Best, Bastian

(57) **Abstract**

Disclosed are methods and systems for creating an avatar (102) of a real-world athlete (104) using artificial intelligence. A corresponding method may comprise providing (802) an avatar (102) corresponding to a real-world athlete (104), receiving (806) athlete feedback (300) from the real-world athlete (104) in response to a behavior of the avatar (102), and adjusting (808) the avatar (102) based on the received athlete feedback (300).

## Description

### TECHNICAL FIELD

The present invention generally relates to the field of computer animation, digitized voice and chat functions, and graphics rendering, and more specifically to techniques for creating avatars of real-world athletes using artificial intelligence.

### BACKGROUND

The advent of technology has brought about a paradigm shift in the way we interact with each other and with digital entities. One fascinating development is the emergence of technology that enables individuals to transform themselves into avatars, such as Synthesia and HeyGen. These platforms offer an intuitive user experience, allowing consumers to create their digital selves by simply uploading videos of themselves.

Moreover, the social media giant Meta has taken this concept a step further by creating celebrity AI chatbots that users can interact with, for instance, through dedicated Instagram accounts of via WhatsApp. One notable example is Sally, Meta's AI chatbot inspired by Australian soccer star Sam Kerr. While Sally boasts Sam Kerr's general visual appearance and tone of voice, the chatbot is designed to provide its own distinct character, engaging with users in conversations about topics including balance, wellness, and nature.

These avatar and AI chatbot solutions represent significant advancements in bridging the gap between reality and the digital world. Users can now engage with digital entities that resemble their favorite personalities and interact with them as if they were real people. Furthermore, these technologies offer novel opportunities for various applications, such as customer service, entertainment, and gaming.

For example, WO 2009/073610 A2 titled "ATHLETIC TRAINING SYSTEM AND METHOD" of Nike Inc. discloses techniques for creating avatars that represent athletes during the replay of an event or as part of a video game. In this document, an avatar may be a virtual representation of appearance of the athlete themselves or may be any other suitable avatar. For example, a viewer may be entertained by replacing the avatar of a star soccer player with the viewer's own avatar. On the other hand, a coach may desire a simpler avatar when analyzing team formations and the like. For example, a football coach may utilize "X" and "O" avatars to represent offense and defense in a football game.

As another example, US 11,130,063 B2 titled "GAMING SYSTEM FOR SPORTS-BASED BIOMECHANICAL FEEDBACK" of Ready 2 Perform Tech LLC discloses a gaming system which allows the user to select a favorite athlete, and then provides biomechanical feedback to the user so that the user may learn to play a particular sport in a manner that mimics the athlete.

Despite the promising advancements in avatar and AI chatbot solutions, there is still room for improvement. Current offerings primarily focus on visual and auditory representations, leaving significant potential untapped and lacking a truly unbiased and genuine representation of the digitalized human counterpart.

It is therefore an objective of the present invention to provide improved techniques for creating avatars, in particular in the sports industry, thereby overcoming the above-mentioned disadvantages of the prior art at least in part.

### SUMMARY OF THE INVENTION

The above-mentioned objective is solved by the subject-matter defined in the independent claims. Advantageous modifications of embodiments of the present invention are defined in the dependent claims as well as in the description and the drawings.

One aspect of the present invention relates to a method of creating an avatar of a real-world athlete. The method may be computer-implemented.

It may be provided that the method comprises a step of providing an avatar corresponding to a real-world athlete. The avatar may be configured to be displayed on a display of an electronic device and/or to speak through a speaker of the electronic device. It may be provided that the avatar is configured to mimic the real-world athlete at least in terms of visual appearance and linguistic expression.

This way, users are enabled to interact with a particularly genuine representation of the real-world athlete, i.e., the avatar, in a more immersive way, as they can see and hear the avatar's responses. This can lead to more engaging and effective interactions, especially when compared to text-based chat interfaces, thereby improving the user experience. The user experience may also be personalized because the avatar can adapt to best suit the user via language, complexity and/or visual representation. In terms of accessibility, especially for users in different age brackets, users with different socio-economic status, users that speak languages other than that of the real-world athlete, users in remote or isolated locations throughout the world, or users with visual or hearing impairments, the ability to interact with an avatar using both visual and auditory channels can significantly enhance the accessibility of these technologies. Moreover, it allows for more seamless interactions with these users as they can engage with the avatars in a way that best suits their abilities. Moreover, avatars displayed on electronic devices with speaking capabilities can facilitate more effective and efficient communication. Furthermore, avatars based on real-world athletes can be used as educational tools, particularly in areas such as sports training and fitness instruction. As will be explained in more detail further below, these avatars can provide personalized guidance and feedback based on their athletic expertise, helping users improve their skills and knowledge.

It may be provided that the avatar is controlled by a machine-learning model that has been trained based on athlete data. The athlete data may comprise at least video data of the real-world athlete.

This way, by training a machine-learning model on video data of real-world athletes, the resulting avatars can exhibit realistic movements and/or expressions that closely resemble their human counterparts. This level of detail and/or accuracy can create a more genuine, engaging and immersive user experience. Furthermore, avatars controlled by machine-learning models can adapt to various situations and contexts, making them more versatile and dynamic. For instance, they can respond differently depending on the user's input or the environment in which they are being used. This ability to learn and adapt makes these solutions more effective and engaging for users. Moreover, machine-learning models used to control avatars can be continuously updated and refined based on new data and user feedback. This ability to learn and adapt over time ensures that these solutions remain up-to-date and effective in meeting the evolving needs of users.

It may be provided that the method comprises a step of receiving athlete feedback from the real-world athlete in response to a behavior of the avatar, and/or a step of adjusting the avatar based on the received athlete feedback. Accordingly, a feedback loop between the real-world athlete and the avatar is provided. The real-world athlete is enabled to provide feedback on the avatar's behavior and/or appearance based on their experience or observation of its performance, and the avatar is adjusted based on the athlete feedback to improve its accuracy, personalization, and effectiveness in representing its human counterpart.

This way, the provided feedback loop helps to create a more accurate, personalized, and/or engaging virtual representation of real-world athletes by incorporating their feedback and, potentially continuously, refining the underlying machine-learning model. This approach offers several advantages, including improved user experience, increased efficiency and enhanced accuracy. First of all, by allowing real-world athletes to provide feedback on the behavior of their virtual counterparts, providers can continuously improve these solutions based on their input. This iterative process ensures that the avatars remain accurate and effective in representing their human counterparts. Receiving athlete feedback and adjusting the avatar accordingly also allows for greater personalization of these solutions. Avatars can be tailored to better replicate the unique movements, expressions, and/or behavior of specific athletes, providing a more engaging and immersive user experience.

It may be provided that the method comprises a step of requesting the athlete feedback from the real-world athlete. It may be provided that the step of requesting the athlete feedback comprises causing a notification on an electronic device of the real-world athlete.

This way, the ability to request athlete feedback directly from real-world athletes using notifications on their electronic devices offers several technical advantages. For example, requesting athlete feedback through notifications on their electronic devices can increase athlete engagement by keeping them informed about the avatar's performance and progress. Athletes may be more inclined to provide feedback if they feel that their input is valued and that it will directly impact their virtual representation. Furthermore, requesting athlete feedback through notifications on their electronic devices provides a convenient way for providers to gather input from athletes, allowing them to do so without the need for face-to-face or other direct interactions. This can save time and resources for both parties while still ensuring accurate and valuable data is collected. Notifications also allow providers to request athlete feedback in real-time and/or at particularly suitable points in time, enabling them to capture data as it occurs and respond promptly to any issues or concerns. This ability to act quickly can help improve the overall user experience and address any potential problems more effectively.

It may be provided that the step of causing a notification on an electronic device of the real-world athlete is based on one or more of: a location of the real-world athlete, an activity level of the real-world athlete, and/or a randomness factor. The location of the athlete may be determined using various methods, including using sensors included in the athlete's electronic device. Common techniques include GPS (Global Positioning System), Wi-Fi triangulation, cellular triangulation, Bluetooth beacons, inertial sensors, geofencing, IP address location, or the like. The activity level of the athlete may be determined using various methods, including using various sensors and/or data points collected from the athlete's electronic device and/or other wearable technology. Some common methods include heart rate monitoring as a reliable indicator of an athlete's physical exertion level. By monitoring heart rate using wearable devices, smartwatches, and/or fitness trackers, it is possible to estimate activity levels based on specific heart rate zones. Another example is using accelerometer data. Accelerometers, which are present in most modern smartphones and wearable devices, can measure the magnitude and direction of forces applied to a device. By analyzing accelerometer data, it is possible to estimate an athlete's activity level based on the intensity and/or duration of movements. For example, sedentary activities like sitting or lying down produce minimal acceleration values, while high-intensity activities like running or jumping result in significant changes in acceleration. Another factor may be Calorie Burning. By estimating the number of calories burned during specific activities based on heart rate, accelerometer data, GPS information, and/or other data points, it is possible to estimate an athlete's overall activity level. For instance, sedentary days with minimal physical exertment produce fewer calories burned compared to active days packed with high-intensity workouts.

This way, by using a combination of factors such as location, activity level, and/or randomness, to determine when to send notifications, providers can tailor their messaging strategy to each athlete's specific context and situation. This may enable providers to engage athletes more effectively by delivering customized, contextually relevant, and timely messages at the right moment. By tailoring their communication strategies to each athlete's unique situation, providers can increase overall user satisfaction and loyalty while still gathering valuable data for enhancing avatars and improving overall user experiences.

Notifications that are sent based on an athlete's location can be used to target athletes when they are in specific environments. This way, providers can capture data at the right moment, enabling them to respond promptly to issues or concerns and improving overall user experience. Location-based notifications allow providers to tailor their messages based on the context of the athlete's current situation. For example, a notification requesting feedback about a particular training session might be more effective if it is sent during that session, when the athlete's focus and attention are dedicated to the activity.

It may be provided that the behavior of the avatar in response to which the athlete feedback is received comprises an artificially created video or audio interview with the avatar. Accordingly, the real-world athlete is presented with a synthetic interview conducted by the avatar, and can provide feedback based on the behavior of the avatar in the interview, such as statements made by the avatar, tone of voice used by the avatar, facial expressions used by the avatar, and the like.

This way, the invention offers an advanced athlete feedback system where the behavior of the avatar is represented through artificially created videos or audios of interviews. One significant technical advantage of this design is that it provides a remarkably realistic impression of how the avatar behaves "in the field". The ability for real-world athletes to see and hear the avatar's interview responses allows them to assess the avatar's behavior in a context that closely resembles actual performance situations. This increased realism significantly improves the accuracy of the feedback provided by the athlete, enabling them to provide particularly suitable feedback based on the observed behaviors, such as statements made, tone of voice used, and facial expressions shown by the avatar. As a result, the invention enhances the overall feedback loop by ensuring that valuable insights are gained from both the real-world athlete's observations and the avatar's responses. This continuous improvement cycle ultimately leads to significant improvements in the quality of the avatar, making it more effective and efficient at providing valuable feedback to athletes for enhanced training and evaluation outcomes.

It may be provided that the behavior of the avatar in response to which the athlete feedback is received comprises an artificially created recording of a sports session with the avatar. Accordingly, the real-world athlete is presented with a recording of the avatar as it performs a sports session, such as a soccer game scene, a set in a weightlifting workout, or the like, and can provide feedback on the behavior of the avatar in the recording.

This way, the invention enables an innovative approach to collecting athlete feedback by providing artificially created recordings of sports sessions featuring an avatar as the primary subject. In this setup, real-world athletes are given the opportunity to review and assess the behavior of the avatar during a simulated sports session. One significant advantage of this design is that it offers a more immersive and realistic experience for the athlete. By providing detailed recordings of the avatar's performance in various sports scenarios, athletes can gain a better understanding of how the avatar behaves under different conditions and circumstances. This improved insight allows for more accurate and relevant feedback to be provided by the real-world athlete, leading to enhanced training and evaluation outcomes.

It may be provided that the athlete data (based on which the machine-learning model that controls the avatar has been trained) comprises sports data of the real-world athlete. The sports data may comprise physiological measurement data of the real-world athlete, in particular at least one of health data or injury data. The sports data may comprise historical sports data of the real-world athlete, in particular sports statistics data. Such sports data may be used in addition or alternatively to the video data described above.

This way, the invention incorporates the real-world athlete's sports data into the machine-learning model that controls the avatar's behavior for more personalized and effective feedback. The sports data may include various types of information, such as physiological measurement data and historical sports statistics data, which can be used to enhance the accuracy and relevance of the avatar's performance. One significant advantage of this design is that it allows for a more customized, personalized, and realistic behavior of the avatar. By incorporating the real-world athlete's sports data into the machine-learning model, the avatar can be trained to simulate behaviors and respond to situations based on the specific characteristics and performance history of the individual athlete.

It may be provided that the video data (as part of the athlete data based on which the machine-learning model that controls the avatar has been trained) comprises athletic video data of the real-life athlete, non-athletic video data of the real-life athlete, or both. The non-athletic video data of the real-life athlete may comprise interview video data and script read-through video data of the real-life athlete.

This way, the invention utilizes a more comprehensive approach to collecting athlete data by being able to incorporate both athletic and non-athletic video data into the machine-learning model that controls the avatar's behavior. The athletic video data may comprise recordings of the real-life athlete participating in sports activities, while the non-athletic video data may comprise interviews and script read-through sessions with the athlete. One significant advantage of this design is that it provides a more holistic understanding of the real-life athlete's behavior patterns and performance characteristics. By analyzing both athletic and non-athletic video data, the machine-learning model can identify correlations between an athlete's verbal communication and their physical performance, enabling the avatar to provide more accurate and personalized behavior. Additionally, the use of interview and script read-through video data offers several other benefits. One such benefit is improved communication and engagement. By incorporating non-athletic video data into the machine-learning model, the avatar can be trained to mimic the real-life athlete's tone, intonation, and body language during interviews, creating a more engaging and relatable avatar. Moreover, in terms of enhanced emotional intelligence, analyzing interview video data can provide insights into an athlete's emotions and psychological state, allowing for more effective and empathetic training recommendations based on their mental well-being. Furthermore, in terms of increased contextual understanding, the use of script read-through video data can help the machine-learning model better understand the nuances and complexities of various sports contexts, enabling the avatar to provide more accurate and relevant behavior in a wider range of situations.

It may be provided that the athlete data comprises audio data of the real-world athlete, in particular one or more of interview audio data and script read-through audio data. This way, a convenient alternative or addition to the above-mentioned video data is provided.

It may be provided that the method comprises a step of providing access to the avatar via an application programming interface. It may be provided that the method comprises a step of providing access to the avatar via a chat interface. It may be provided that the method comprises a step of providing access to the avatar via a coaching application. The various ways of providing access to the avatar may be combined.

This way, the invention offers multiple options for accessing and interacting with the avatar, catering to various user preferences and requirements. The methods described below outline these different means of access.
1. Application Programming Interface (API) Access: The method may involve providing a developer-friendly interface that enables third-party applications to easily integrate the avatar's functionality into their software solutions. This allows for seamless and automated interactions with the avatar, enabling developers to create custom applications such as training programs, performance evaluations, or other applications that leverage the avatar.
2. Chat Interface Access: The method may offer a user-friendly text-based chat interface where users can interact with the avatar in real-time. This approach is ideal for individuals who prefer a more straightforward and conversational interaction style, as well as for situations where visual or audio feedback may not be practical or accessible.
3. Coaching Application Access: The method may include making the avatar available through a dedicated coaching application that offers a more comprehensive and feature-rich experience. This app may include advanced analytics tools, customizable training plans, progress tracking, and social networking capabilities to help users optimize their performance and connect with other athletes and coaches.

By providing access to the avatar via one or more of these interfaces, the invention ensures that it remains versatile and adaptable to various user needs and preferences, ultimately increasing its utility and reach in the world of sports training and evaluation.

Another aspect of the present invention relates to a method of athletic performance feedback by an avatar of a real-world athlete. The method may comprise a step of providing an avatar corresponding to a real-word athlete that has been created in accordance with any of the methods described herein. The method may comprise a step of receiving a video recording of an athletic activity of a user. The method may comprise a step of providing athletic performance feedback to the user in a video in which the avatar of the real-world athlete is commenting on the athletic activity of the user.

This way, by offering athletic performance feedback via an avatar of a real-world athlete, the invention provides users with more accurate, personalized, and relatable guidance tailored to their specific sports activities and goals. This approach fosters increased engagement, motivation, and improved overall performance in various athletic pursuits. When the avatar is built based on a real-life athlete who is a professional in the relevant sport or a celebrated figure, the athletic performance feedback system offers several advantages. In terms of authenticity and credibility, feedback from a avatar of a well-known athlete adds an element of authenticity and credibility to the training and evaluation process. Users can trust that they are receiving guidance and recommendations from someone with extensive experience and a proven track record of success in their chosen sport or activity. In terms of inspiration and motivation, interacting with an avatar based on a professional athlete or celebrity provides users with an increased sense of inspiration and motivation. Seeing and learning from the experiences and achievements of these individuals can help users stay focused on their own goals and progress, as well as foster a deeper connection to their chosen sport or activity. In terms of personalized and targeted feedback, the advanced machine-learning algorithms used in the system can analyze user performance data in greater detail when dealing with professional athletes or celebrities' avatars. This results in more accurate, targeted, and effective feedback tailored to individual users and their specific athletic needs, helping them to make faster improvements and achieve better overall performance. In terms of enhanced learning opportunities, interacting with an avatar of a professional athlete or celebrity allows users to gain valuable insights into the techniques, strategies, and mindset required to excel in their chosen sport or activity. This knowledge can help users refine their skills, broaden their understanding of the sport, and ultimately reach their full potential.

Another aspect of the present invention relates to a data processing apparatus. The data processing apparatus may comprise means for carrying out a method according to any one of the aspects described herein. Another aspect of the present invention relates to a data processing apparatus comprising a memory and one or more processors coupled to the memory, the one or more processors being configured to carry out any of the methods described herein. A data processing apparatus may comprise any kind of data processing hardware and may encompass all kinds of apparatus, devices, and machines for processing data, including by way of example a programmable processor, a computer, or multiple processors or computers.

Another aspect of the present invention relates to a computer program. Another aspect of the present invention relates to a computer-readable medium having stored thereon a computer program. The computer program may comprise instructions which, when the program is executed by a computer, cause the computer to carry out a method according to any one of the aspects described herein. A computer program may also be referred to as a program, software, a software application, an app, a module, a software module, a script, or code. A computer program may be written in a programming language, including compiled or interpreted languages. A computer program may be deployed in any form, including as a stand-alone product or as a module, component, subroutine, or other unit suitable for use in a computing environment.

Another aspect of the present invention relates to a non-transitory computer-readable medium storing a set of instructions that, when executed by one or more processors of an apparatus, cause the apparatus to carry out any of the methods described herein.

The terms used herein should generally be construed as understood by the average person skilled in the art, unless explicitly indicated otherwise. The following explanations may guide the understanding:
The term "real-world athlete", or "athlete" in short, should be understood as a person engaged in a particular kind of sport, such as cricket, soccer, basketball, American football, baseball, or the like. The athlete may be professional athlete.

The term "avatar" should be understood as a virtual or digital representation of a person, the person's character, and/or persona. An avatar may comprise a graphical representation, in particular in the form of a three-dimensional model. An avatar may be usable in digital worlds, video games, as well as other online settings including social media, virtual assistants, and instant messaging platforms.

The term "artificial Intelligence" (AI) should be understood as referring to a branch of computer science that aims to develop machines or software capable of intelligent behavior, typically with the goal to mirror or surpass human intelligence in specific tasks. AI systems are designed to perform complex tasks such as reasoning, learning, perception, problem-solving, and understanding natural language. These systems can typically adapt to new situations and improve their performance over time. The goal of AI is to create systems that can function autonomously and interact with their environment in a human-like manner.

The term "machine learning" (ML) should be understood as a subset of artificial intelligence that focuses on the development of algorithms and statistical models that enable computers to perform specific tasks without using explicit instructions. Instead, machine-learning systems learn and make predictions or decisions based on data. Machine-learning algorithms build a mathematical model based on sample data, known as training data, to make predictions or decisions without being explicitly programmed to perform the task. Machine learning can be employed in a variety of applications, including image and speech recognition, medical diagnosis, predictive analytics, and many more, where it enables systems to learn from and adapt to new data independently.

The term "machine-learning algorithm" should be understood as a computational procedure that is designed to analyze data, learn from it, and identify patterns or make decisions based on the input data without being explicitly programmed for the task. Machine-learning algorithms leverage statistical techniques to enable systems to improve their performance on a specific task with more data over time. Machine-learning algorithms are the foundation upon which machine-learning models are built, providing the methods or processes through which data is transformed into actionable insight. Examples of machine-learning algorithms include linear regression, decision trees, support vector machines, and neural networks, among others.

The term "machine-learning model" should be understood as referring to the output generated when a machine-learning algorithm is trained on a dataset. It represents the knowledge or understanding gained by the algorithm from the data, encapsulating the learned patterns or predictions. Essentially, a machine-learning model is what enables predictions or decisions based on new, unseen data, based on the learning it has derived from the training process. The machine-learning model is typically defined by its parameters, which may be adjusted during the training phase to minimize the difference between the predicted outcome and the actual outcome. Although, strictly speaking, "machine-learning algorithm" and "machine-learning model" have distinct definitions, it is not uncommon for these terms to be used interchangeably in casual discourse. This usage stems from the close relationship between algorithms and models in the workflow of machine-learning projects, where the algorithm is the means of creating the model. Therefore, these terms may be used synonymously herein unless the distinction is decisive.

The term "artificial neural network" (ANN), or "neural network" (NN) in short, should be understood as a machine-learning or deep-learning model or algorithm. Neural networks are generally inspired by the human brain and typically comprise interconnected nodes or neurons organized into layers. Neural networks can be used to process data and learn from examples, enabling them to perform tasks such as image recognition, natural language processing, and more. A neural network typically comprises an input layer, one or more hidden layers, and an output layer. Through a process called training, neural networks can learn to perform specific tasks by adjusting their internal parameters, or "weights", based on labeled or unlabeled data.

The term "training" should be understood as referring to the process of teaching a machine-learning model to make predictions or decisions, by exposing it to data for which the outcomes are known. The training process typically involves feeding a training dataset into a machine-learning algorithm, which then uses statistical analysis to learn the patterns or relationships within the data. During training, the algorithm iteratively adjusts the parameters of the model to minimize the difference between the predicted outcomes and the actual outcomes in the training data. This adjustment process is typically guided by a loss function, which measures the accuracy of the model's predictions. The goal of training is to produce a model that accurately represents the underlying structure of the data, enabling it to make reliable predictions about new, unseen data. Supervised learning involves training a model on a labeled dataset, where each example in the training data is paired with the correct output. The model learns to predict the output from the input data. Unsupervised learning involves training a model on data without labeled responses. The model tries to find patterns and relationships in the data on its own. Semi-supervised learning combines both labeled and unlabeled data during the training process, which can be beneficial when acquiring a fully labeled dataset is costly or impractical.

The term "large language model" (LLM) should be understood as referring to a type of machine-learning model that has been trained to recognize, generate, translate, and/or summarize vast quantities of written human language and textual data. LLMs are notable for their ability to achieve general-purpose language generation. LLMs comprise a large number of parameters, typically in the millions or often billions of parameters, which enable them to capture a wide array of linguistic nuances, patterns, and contexts.

### BRIEF DESCRIPTION OF THE DRAWINGS

The invention may be better understood by reference to the following drawings:
- Fig. 1:: A schematic block diagram of a system in accordance with embodiments of the invention
- Fig. 2:: A schematic overview of a taxonomy of athlete data in accordance with embodiments of the invention
- Fig. 3:: A flow diagram of a high-level overview of an exemplary workflow in accordance with embodiments of the invention
- Fig. 4:: Exemplary user interface screens for implementing an Athlete Permission function in accordance with embodiments of the invention
- Fig. 5:: Exemplary user interface screens for implementing an Athlete Inputs function in accordance with embodiments of the invention
- Fig. 6:: A schematic overview of a model creation and training architecture in accordance with embodiments of the invention
- Fig. 7:: Exemplary user interface screens for implementing an Athlete Authentication function in accordance with embodiments of the invention
- Fig. 8:: A flow diagram of a method of creating an avatar in accordance with embodiments of the invention
- Fig. 9:: A flow diagram of a method of athletic performance feedback by an avatar in accordance with embodiments of the invention
- Fig. 10:: A block diagram of a system architecture in accordance with embodiments of the invention
- Fig. 11:: A flow diagram of an end-to-end workflow in accordance with embodiments of the invention
- Fig. 12:: A block diagram of an athlete portal in accordance with embodiments of the invention

### DETAILED DESCRIPTION

In the following, representative embodiments illustrated in the accompanying drawings will be explained. It should be understood that the illustrated embodiments and the following descriptions refer to examples which are not intended to limit the embodiments to one preferred embodiment.

Certain embodiments provide for the creation of authentic avatars, also referred to as "AI Athletes" based on one or both of the following: Engaging athletes to gain their permission, inputs and approval, and producing avatars that are powered by, and constantly evolving based on, athlete inputs and sports data, and therefore have the sporting ability and intelligence of the athlete they represent.

Certain embodiments may utilize athlete feedback loops to create and continually improve the personalities and sporting traits of AI Athletes. The described embodiments may use athlete data, e.g., including match statistics, health data, physical measurement data, injuries, etc., potentially combined with the latest news and interviews given by and about a real-life athlete to train an avatar to represent the real-life athlete's personality, physical attributes and/or skills. This along with a historical database of their playing statistics and/or achievements for concrete references, when needed, combine to become a powerful API which can be integrated into voice and/or avatar assets and which can be made accessible to third-party companies to implement for their own voice and/or avatar assets.

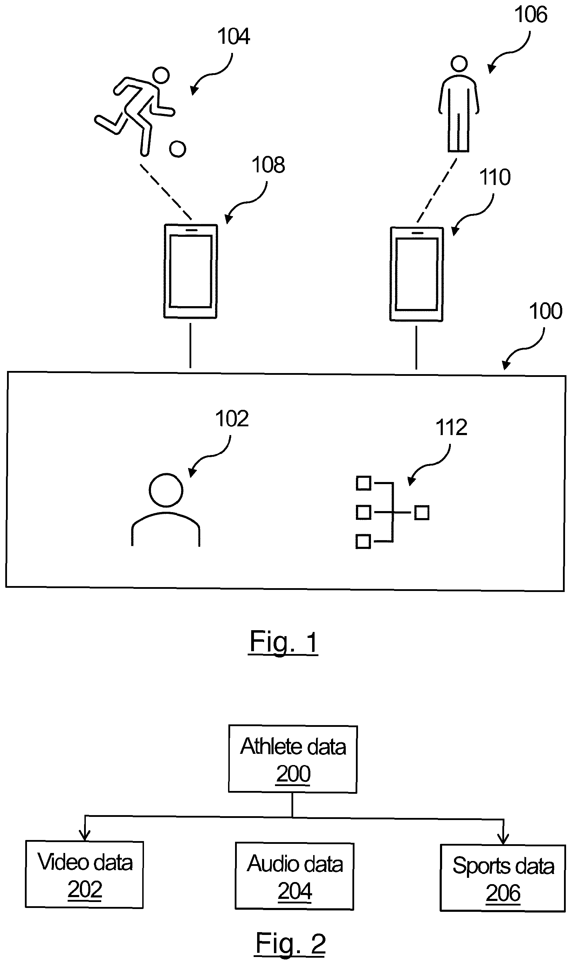

Fig. 1 illustrates a schematic block diagram of a system 100 in accordance with an exemplary embodiment. The system 100 comprises an avatar 102 controlled by a machine-learning model 112. The avatar 102 has been trained to mimic a real-world athlete 104 who can interact with the system using their electronic device 108. Once trained, the avatar 102 can be accessed by users 106 through their electronic devices 110.

Fig. 2 illustrates a schematic overview of a taxonomy of athlete data 200 in accordance with an exemplary embodiment. As can be seen, the athlete data 200 can comprise video data 202, audio data 204, sports data 206, or any combinations thereof.

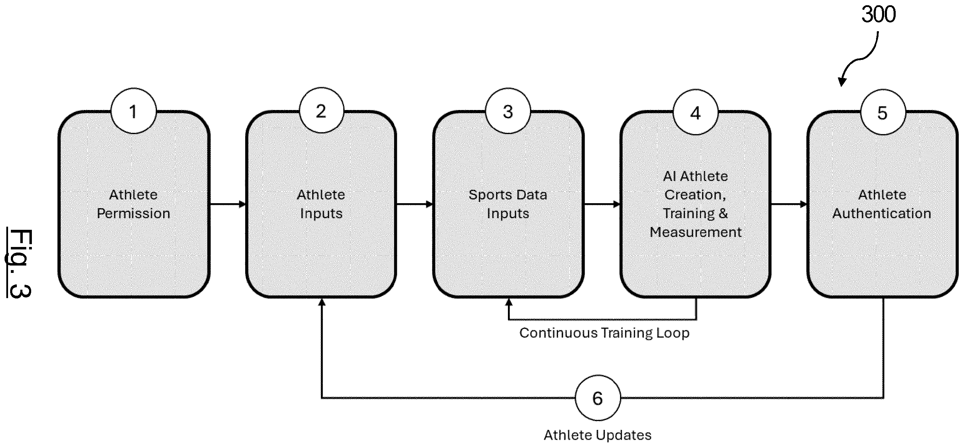

Fig. 3 illustrates a high-level overview of an exemplary workflow 300 in accordance with certain embodiments. The workflow 300 proceeds through the phases "Athlete Permission" (phase 1 in Fig. 3), "Athlete Inputs" (phase 2), "Sports Data Inputs" (phase 3), "AI Athlete Creation, Training & Measurement" (phase 4) and "Athlete Authentication" (phase 5). A continuous training loop between phases 4 and 3 is also depicted. In phase 6, Athlete Updates are performed. Each of these phases will be explained in more detail in the following:

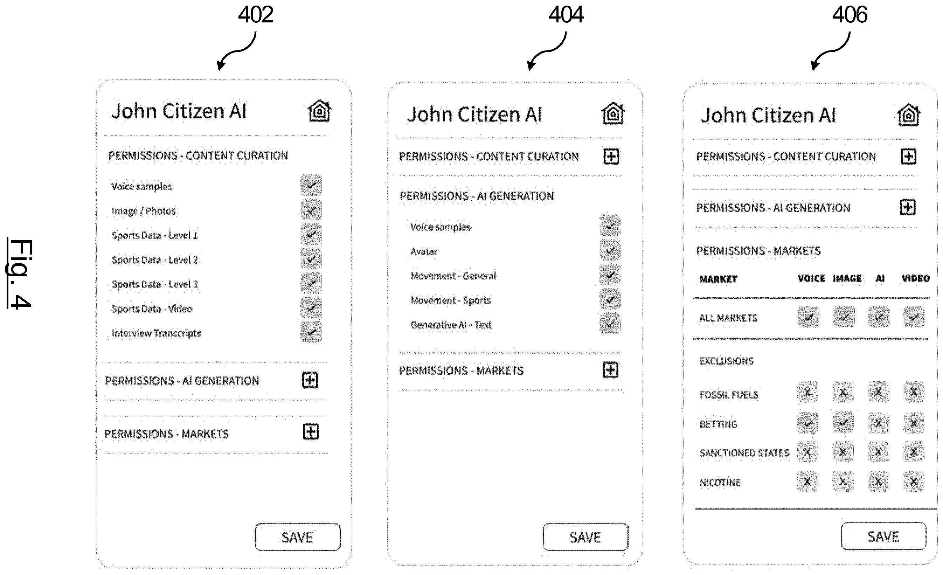

Fig. 4 illustrates exemplary user interface screens for implementing an Athlete Permission function in accordance with certain embodiments. The Athlete Permission function may be carried out in phase 1 of Fig. 3. In screen 402, the athlete 104 can control the collection of the materials, also referred to as athlete data 200, used to create their AI Athlete 102. As illustrated, the materials may comprise voice samples, images, photos, video data, sports data, audio data, interview transcripts, or any combination thereof. In screen 404, the athlete 104 can control the generation of the modules that comprise their AI Athlete 102. As illustrated, the modules may comprise voice samples, avatar, general movement, sports movement, text, or any combination thereof. In screen 406, the athlete 104 can control the use and/or commercialization of their AI Athlete 102 in various markets. As illustrated, the athlete 104 may also exclude certain topics, such as fossil fuels, betting, sanctioned states, nicotine, or any combination thereof.

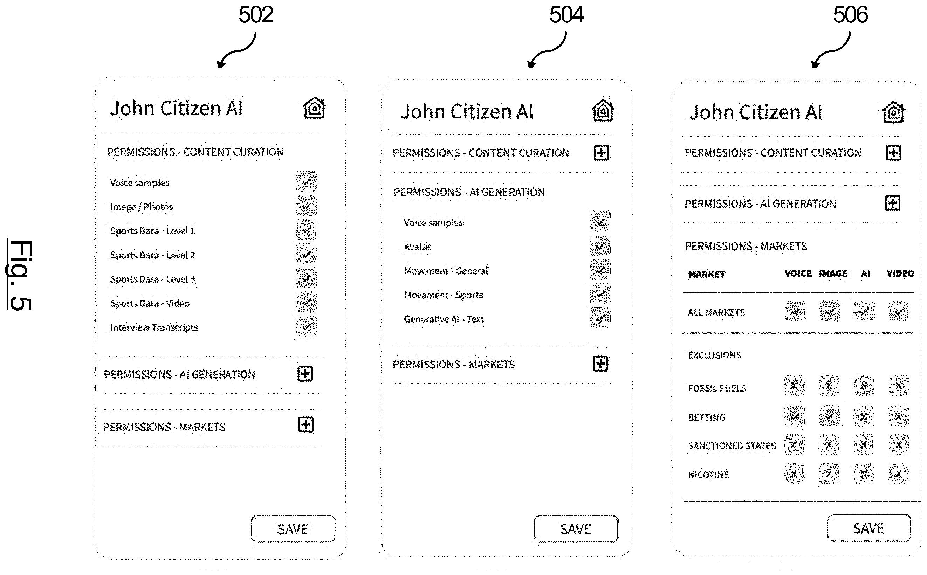

Fig. 5 illustrates exemplary user interface screens for implementing an Athlete Inputs function in accordance with certain embodiments. The Athlete Inputs function may be carried out in phase 2 of Fig. 3. In screen 502, the athlete 104 can provide personal inputs to support the creation of their AI Athlete 102. In screen 504, the athlete 104 can provide voice inputs, such as by reading a text displayed on the screen. In screen 506, the athlete 104 can control the likeness of their AI Athlete 102.

In phase 3 "Sports Data Inputs" of Fig. 3, sports data can be sourced and/or utilized to supplement the personal inputs that the athlete 104 has provided. The inputs may comprise one or more of the following:
L0 - comps: Fixture data and/or historical match statistics can be sourced from a third-party supplier such as ESPN Cric Info. This information can be ingested and stored via an API integration.

L1 - event data (shot type, etc.): Detailed event data (e.g., per ball) can be sourced from a third-party supplier such as Cricket21. This information can be ingested and stored via an API integration.

L2 - ball tracking: Data can be sourced, e.g., from Hawkeye or a third-party CV technology company like Gameface.ai can be used to go over historical and/or live footage. This information can be ingested and stored via an API integration.

L3 - skeletal data: Pose detection data can be gathered by collecting video footage of the athlete 104 and running a pose detection CV model to extract the data.

Health data: This data can be supplied by the athlete 104 or on behalf of the athlete 104 by an organization that already has access to it.

Video and/or interviews: Public domain data can be extracted from the Internet, video and/or interviews that the athlete 104 has access to.

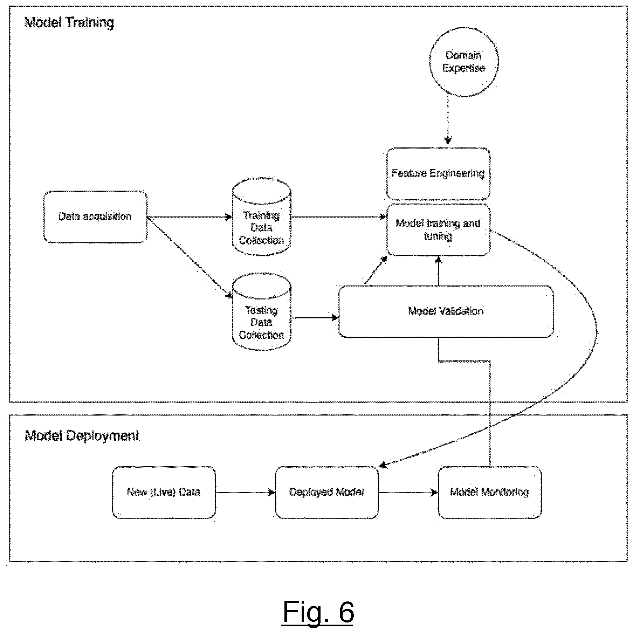

In phase 4 "AI Athlete Creation, Training & Measurement" of Fig. 3, a process to create and train the AI model can be carried out. During model training, algorithm designers can collect a training dataset comprising data from different modalities, each acquired according to some predefined protocol. These data can be used to engineer a feature set and train a model to automate a decision of interest. The final model and feature set can be selected using a cross-validation procedure on a held-out test set. After model deployment, real-world model performance can be monitored, and the original model can be iteratively updated and redeployed. Fig. 6 illustrates a schematic overview of a suitable model creation and training architecture.

Certain embodiments may also comprise a process to measure the models (e.g., against optimal benchmark). The various models used to combine to create the AI Athlete 102 may have different ways to measure them. The following are examples:
Voice model: By taking regular voice recordings from the athlete 104, wave forms of generated voice audio can be compared using the same words to compare the accuracy.

Image model: The athlete 104 may be asked, e.g., periodically, for image feedback from the athlete 104 as to whether they believe the created AI images look like them.

Individual sport performance: Using data streams of real-world matches, athlete models can be tested against decisions they made to see how the AI model responds. Various factors will be used to measure the accuracy.

Team based sporting tactics: Using data streams and/or results of real-world matches, the models can be tested to see virtualized outcomes versus real-world outcomes.

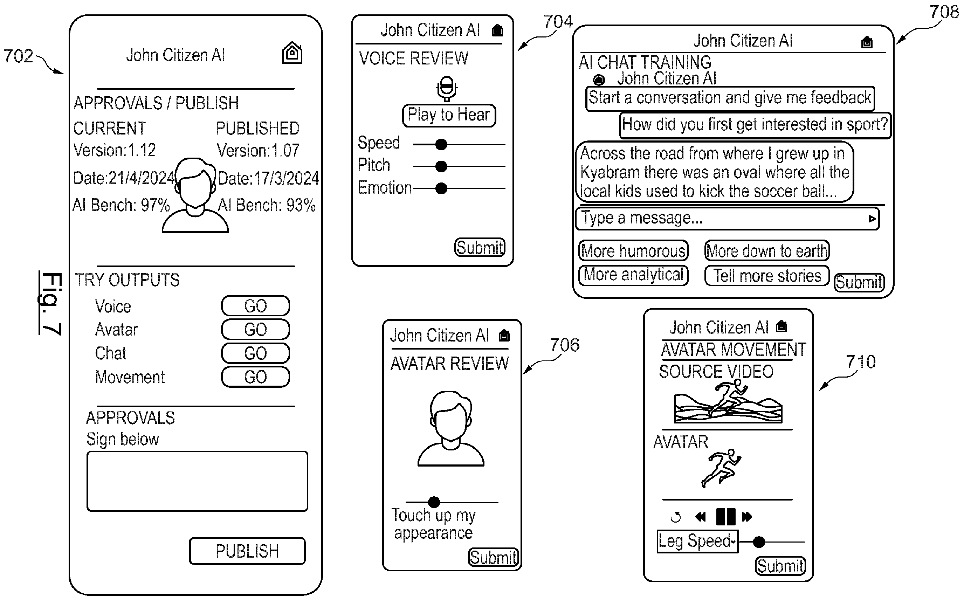

Fig. 7 illustrates exemplary user interface screens for implementing an Athlete Authentication function in accordance with certain embodiments. The Athlete Authentication function may be carried out in phase 5 of Fig. 3. In screen 702, the athlete 104 can review and/or approve their AI Athlete 102.

In certain embodiments, the review can be conducted on a per module basis, e.g., per voice (screen 704), likeness/avatar (screen 706), chat (screen 708), sports skill and/or movement (screen 710), or any combination thereof.

In certain embodiments, the approval can be provided via electronic signature. This then authenticates the AI Athlete 102, and optionally tracks the version (e.g., via blockchain or other measures).

In phase 6 "Athlete Updates" of Fig. 3, the athlete 104 can continually review and/or update their AI Athlete 102, e.g., by providing additional and/or updated data (such as voice, likeness, uploaded content, or any other form of data). This may be accomplished by using different screen states of the Athlete Inputs screen explained above.

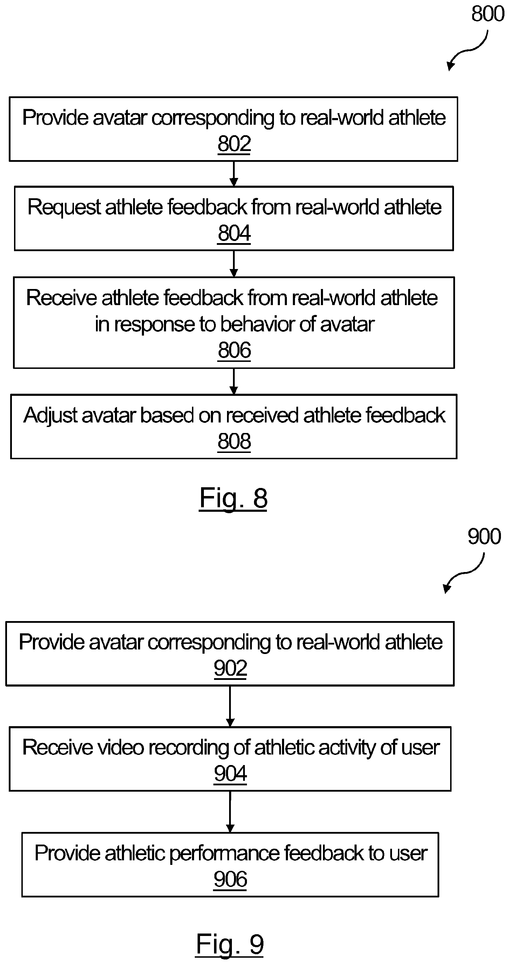

Fig. 8 illustrates a flow diagram of a method 800 of creating an avatar 102 in accordance with embodiments of the invention. The method 800 may be practiced using the functions explained above. An avatar 102 corresponding to a real-world athlete 104 is provided in step 802. The avatar 102 may be configured to be displayed on a display of an electronic device 108, 110 and to speak through a speaker of the electronic device 108, 110. The avatar 102 may be configured to mimic the real-world athlete 104 at least in terms of visual appearance and linguistic expression. The avatar 102 may be controlled by a machine-learning model 112 that has been trained based on athlete data 200. The athlete data 200 may comprise at least video data 202 of the real-world athlete 104. Athlete feedback 300 may be requested from the real-world athlete 104 in step 804. Athlete feedback 300 may be received from the real-world athlete 104 in response to a behavior of the avatar 102 in step 806. The avatar 102 may be adjusted based on the received athlete feedback 300 in step 808.

Fig. 9 illustrates a flow diagram of a method 900 of athletic performance feedback by an avatar 102 of a real-world athlete 104 in accordance with embodiments of the invention. An avatar 102 corresponding to a real-word athlete 104 that has been created in accordance with the method 800 may be provided in step 902. A video recording of an athletic activity of a user 106 may be received in step 904. Athletic performance feedback may be provided to the user 106 in step 906, e.g., in a video in which the avatar 102 of the real-world athlete 104 is commenting on the athletic activity of the user 106.

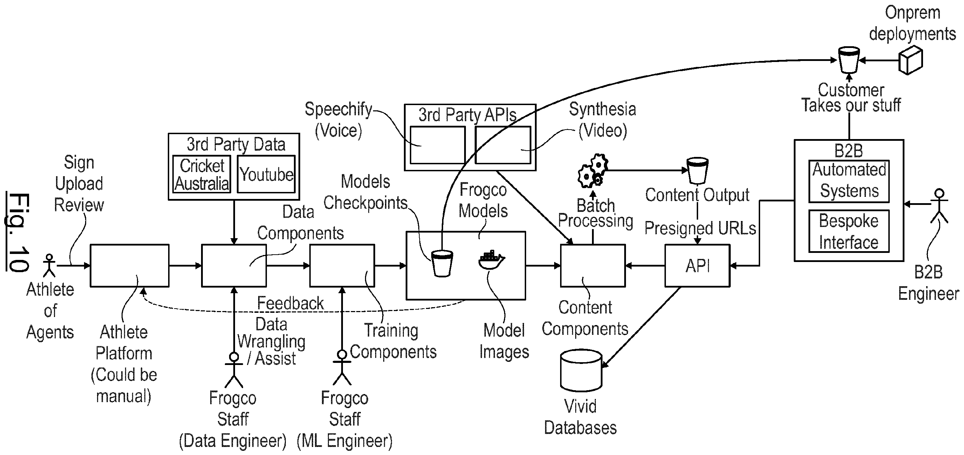

Fig. 10 illustrates a block diagram of a system architecture in accordance with certain embodiments of the invention.

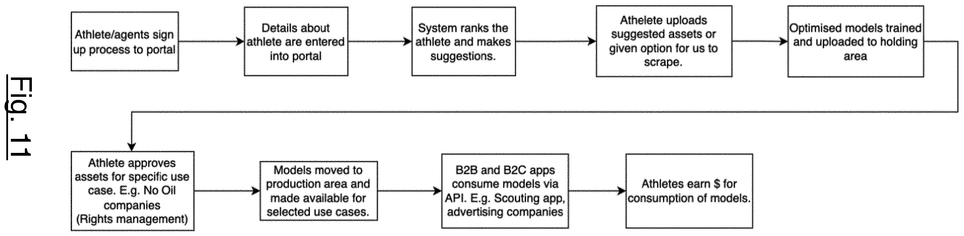

Fig. 11 illustrates a flow diagram of an end-to-end workflow from an avatar being configured in the system to a consumer using the produced avatar in accordance with embodiments of the invention. The workflow may comprise one or more of the following:
- Athlete signs up to the portal.
- Athlete enters details about themselves.
- Interface provides feedback about what assets to provide or option to have system scrape.
- Athlete uploads assets.
- System trains models and produces previews for athlete.
- Athlete approves for use.
- Third-party applications can now use model based on criteria.
- Athlete gets paid.

From the perspective of a B2B user, the system may be used as follows in one exemplary embodiment:
- Business signs up as a consumer
- Business user is given authority to access specific models
- Business is given an API key and documentation to interface
- Business makes calls to the API to produce model output. For example, a voice of an athlete saying something specific.
- There may be a portal that the business can access to make further configuration changes.

From the perspective of a B2C user, the system may be used as follows in one exemplary embodiment:
- App user registers to mobile app
- App user subscribes to mobile app
- App user selects famous athlete to have them train them, and/or uploads video footage for scouting, and/or watches and shares own video content with other users/ selects automated commentary from AI athlete of choice.
- End user can search for "scoutable" athletes.
- Mobile app makes calls to API to produce appropriate assets
- End user sees content in the form of their favourite athlete.

From the perspective of a real-life athlete, the system may be used as follows in one exemplary embodiment:
- App for athlete to login and see/hear their AI
- Give feedback in a light-touch manner

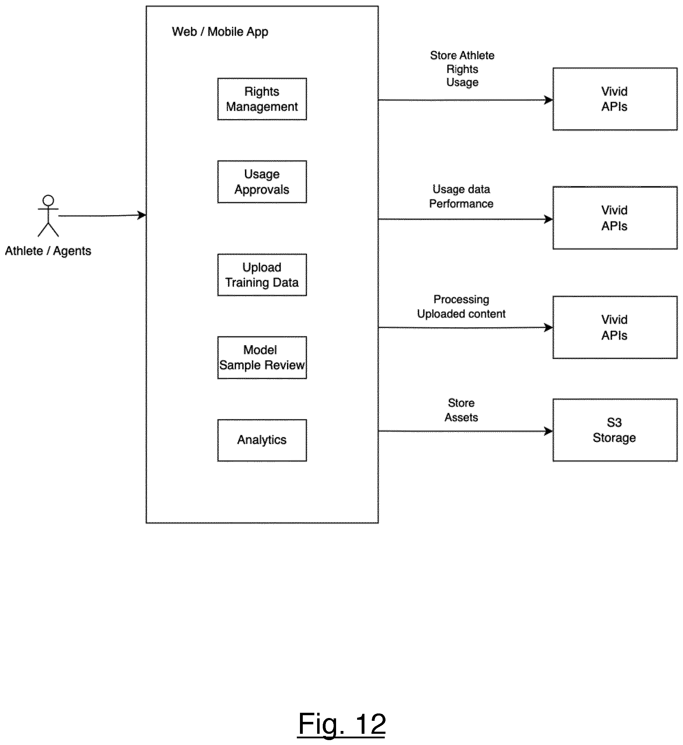

Fig. 12 illustrates an athlete portal in accordance with certain embodiments of the invention. In the following, an exemplary implementation of aspects of such an athlete portal in accordance with embodiments of the invention will be described.

The Athlete portal may be written in HTML and JavaScript using the React framework. It may be deployed to a Netlify and accessible via a domain name. Athletes or agents may sign in to this portal using a central authentication system.

Interfaces into the platform may be serviced by Python-powered APIs. These may run as docker containers in Kubernetes and may be scaled as demand requires. The frontend portal may use these to provide feedback to the user. The consumer interfaces may use these to retrieve output assets. The internal system may also use APIs to pass data required for the system to work.

A PostgreSQL database may be provisioned to store relational data. For example, information about the athlete, models available, ranking information, analytics, etc. All API components can interface with this database.

AWS S3 or a similar cloud object storage may be used for storing models and assets. For example, when a consumer wants to produce an output voice clone of an athlete, the model may be brought down into a docker container where the output is produced and re-uploaded to an S3 location. The consumer application may be then given access to retrieve it via API calls.

The AWS Message Queuing service SQS may be used to send messages between different components of the system. This may be particularly important for batch processing. For example, when an athlete uploads assets for model development, it should be made sure the system is not overwhelmed, and it can be controlled when model development jobs are collected and processed.

Kubernetes may be used to orchestrate processing components. Different parts of the system may be serviced out of docker containers and scaled out using Kubernetes. For example, scaling out model training processes.

Python-based model training frameworks such as Pytorch may be used to produce optimized models that may subsequently be used for inference. These may be incorporated into docker images that are deployed to the Kubernetes cluster for processing. There may be different ones depending on the problem and media type, for example by incorporating voice cloning via TTS (https://github.com/coqui-ai/tts) and/or a CycleGAN based approach for realistic face swaps.

Some of these may be containers with code to interface with third party products that provide APIs such as Speechify and D-ID.

Mobile apps may be programmed in React Native and Expo. They may use the consumer APIs to access Athlete models, for example, for providing text and getting back audio of an Athlete. Other consumers may use the API to produce mobile or web apps.

In summary, certain embodiments of the invention as disclosed herein utilize athlete feedback loops to create and/or continually improve the personalities and/or sporting traits of AI Athletes 102. This may enable time-poor athletes 104 to give feedback on the performance of their own AI Athlete 102, thereby enabling efficient, scalable and continual learning by the AI Athlete 102. The resulting AI Athlete 102 may comprise one or more of the following characteristics:
- Speak like the athlete 104, i.e., voice sounds the same
- Looks like the athlete 104, i.e., has a face and/or body represented as a digital avatar
- Can answer questions in the way the athlete 104 would, e.g., via voice and/or text
- Can perform sports skills like the athlete 104
- Can demonstrate and explain to a user 106 how to perform skills better

Certain embodiments of the invention can be used in the following applications:
- Computer Gaming Industry, e.g., for companies and developers looking to integrate realistic athlete Als into games, enhancing character and gameplay styles
- Advertisers, e.g., companies seeking sophisticated tools for audience identification and targeted advertising, especially in sports
- Media Companies, e.g., organizations involved in broadcasting and digital media, utilizing AI for content creation and enhancement
- Sports Performance and Coaching Entities, e.g., professional sports teams and coaching institutions interested in virtual coaching and scouting tools
- Athletes and Sports Leagues, such as Bundesliga, managing their digital rights and leagues looking to engage fans and monetize content
- Broadcasters and Rights Holders, e.g., entities owning or distributing sports content, seeking to leverage AI for enhanced viewer experiences
- Players' unions, such as Federation of International Cricketers' Associations (FICA) specifically partnering for revenue generation from AI applications
- Business-to-Business (B2B) Clients, e.g., companies using the platform's API Integration systems for various commercial applications

Certain embodiments of the invention may overcome, without limitation, one or more of the following limitations of the prior art:
- Limited Realism in Computer Gaming Characters: Embodiments of the invention may provide highly realistic AI-generated athletes, enhancing the authenticity of sports games and player experience.
- Ineffective Targeted Advertising: Embodiments of the invention may offer advanced tools for audience identification and targeting in advertising, ensuring more effective marketing campaigns tailored to regional preferences and trends.
- Content Creation Challenges in Media: Embodiments of the invention may assist media companies in creating engaging and innovative content, utilizing AI to generate new forms of athlete-focused media.
- Resource Constraints in Coaching and Scouting: Embodiments of the invention may introduce virtual coaching and scouting tools, making athlete training and talent identification more accessible and less resource intensive.
- Athlete Brand Management: Embodiments of the invention may help athletes manage their digital rights and personal brand, providing a platform for them to control and monetize their likeness and content.
- Data Management and Analytics: Embodiments of the invention may provide a robust system for securely processing and storing diverse data sources, along with detailed analytics for better decision-making.
- Diverse Revenue Generation Needs: Embodiments of the invention may address the varied revenue models of different stakeholders (like athletes, leagues, and broadcasters) through flexible commercial arrangements like revenue sharing.
- Fan Engagement and Interaction: Embodiments of the invention may enhance fan experiences by offering interactive and personalized sports content, deepening fan loyalty and engagement.
- Market and Technology Evolution: Embodiments of the invention may help stay ahead of rapidly changing market trends in athlete commercialization and fan experiences, leveraging the latest in generative AI technology.
- Fundamentally biased AI models: Embodiments of the invention and its use of athlete engagement and feedback may circumvent foundational bias in AI models caused by the use of biased, underlying LLMs.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Embodiments of the invention may be implemented on a computer system. The computer system may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system may comprise any circuit or combination of circuits. In one embodiment, the computer system may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), or any other type of processor or processing circuit. Other types of circuits that may be included in the computer system may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system may also include a display device, one or more speakers, and a keyboard and/or controller, which can include a mouse, trackball, touch screen, voice-recognition device, or any other device that permits a system user to input information into and receive information from the computer system.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitory. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

## Claims

1. A method (800) of creating an avatar (102) of a real-world athlete (104), comprising:
(a) providing (802) an avatar (102) corresponding to a real-world athlete (104);
(i) wherein the avatar (102) is configured to be displayed on a display of an electronic device (108; 110) and to speak through a speaker of the electronic device (108; 110);
(ii) wherein the avatar (102) is configured to mimic the real-world athlete (104) at least in terms of visual appearance and linguistic expression;
(b) wherein the avatar (102) is controlled by a machine-learning model (112) that has been trained based on athlete data (200);
(i) wherein the athlete data (200) comprises at least video data (202) of the real-world athlete (104);
(c) receiving (806) athlete feedback (300) from the real-world athlete (104) in response to a behavior of the avatar (102); and
(d) adjusting (808) the avatar (102) based on the received athlete feedback (300).

2. The method of claim 1, comprising: requesting (804) the athlete feedback (300) from the real-world athlete (104).

3. The method of claim 2, wherein the step of requesting (804) the athlete feedback (300) comprises causing a notification on an electronic device (108) of the real-world athlete (102) based on one or more of:
- a location of the real-world athlete (104);
- an activity level of the real-world athlete (104);
- a randomness factor.

4. The method of any one of the preceding claims, wherein the behavior of the avatar (102) in response to which the athlete feedback (300) is received comprises:
- an artificially created video or audio interview with the avatar (102);
- an artificially created recording of a sports session with the avatar (102).

5. The method of any one of the preceding claims, wherein the athlete data (200) comprises sports data (206) of the real-world athlete (104).

6. The method of claim 5, wherein the sports data (206) comprises physiological measurement data of the real-world athlete (104), in particular at least one of health data or injury data.

7. The method of claim 5 or 6, wherein the sports data (206) comprises historical sports data of the real-world athlete (104), in particular sports statistics data.

8. The method of any one of the preceding claims, wherein the video data (202) comprises athletic video data of the real-life athlete (104).

9. The method of any one of the preceding claims, wherein the video data (202) comprises non-athletic video data of the real-life athlete (104), in particular one or more of: interview video data and script read-through video data.

10. The method of any one of the preceding claims, wherein the athlete data (200) comprises audio data (204) of the real-world athlete (104), in particular one or more of: interview audio data and script read-through audio data.

11. The method of any one of the preceding claims, comprising:
providing access to the avatar (102) via an application programming interface; and/or
providing access to the avatar (102) via a chat interface; and/or
providing access to the avatar (102) via a coaching application.

12. A method (900) of athletic performance feedback by an avatar (102) of a real-world athlete (104), comprising:
(a) providing (902) an avatar (102) corresponding to a real-word athlete (104) that has been created in accordance with the method (800) of any one of claims 1-11;
(b) receiving (904) a video recording of an athletic activity of a user (106); and
(c) providing (906) athletic performance feedback to the user (106) in a video in which the avatar (102) of the real-world athlete (104) is commenting on the athletic activity of the user (106).

13. A data processing apparatus comprising means for carrying out the method of any one of claims 1-12.

14. A computer program or a computer-readable medium having stored thereon the computer program, the computer program comprising instructions which, when the program is executed by a computer, cause the computer to carry out the method of any one of claims 1-12.
